# EUROPÄISCHE PATENTSCHRIFT

(11) **EP 1 272 447 B1**
(45) Veröffentlichungstag und Bekanntmachung des Hinweises auf die Patenterteilung: **02.11.2005**
(21) Anmeldenummer: 01927751.6
(22) Anmeldetag: 19.03.2001
(51) Int. Cl.: C07C 17/12, C07C 25/13

(54) **VERFAHREN ZUR HERSTELLUNG VON 2,3,4,5-TETRACHLORBENZOTRIFLUORID**
METHOD FOR PRODUCING 2,3,4,5-TETRACHLOROBENZOTRIFLUORIDE
PROCEDE DE PREPARATION DE 2,3,4,5-TETRACHLOROBENZOTRIFLUORURE

(30) Priorität: 31.03.2000 DE 10016192
(43) Veröffentlichungstag der Anmeldung: 08.01.2003
(73) Patentinhaber: LANXESS Deutschland GmbH, 51369 Leverkusen (DE)
(72) Erfinder: MAIS, Franz-Josef, 40591 Düsseldorf (DE); LAHR, Helmut, 51519 Odenthal (DE)
(86) Internationale Anmeldenummer: PCT/EP2001/003112
(87) Internationale Veröffentlichungsnummer: WO 2001/074746

(56) Entgegenhaltungen:
- DE-A- 4 301 247
- US-A- 4 401 623
- US-A- 5 750 811
- USHAKOV A A ET AL: "Features of the high chlorination of 4-chloro- and 2,4-dichlorotrifluoromethylbenzenes" JOURNAL OF ORGANIC CHEMISTRY OF THE USSR. (ZHURNAL ORGANICHESKOI KHIMII), Bd. 20, Nr. 10, 1984, Seiten 1993-1996, XP002171256 CONSULTANTS BUREAU. NEW YORK., US in der Anmeldung erwähnt

## Beschreibung

Die vorliegende Erfindung betrifft ein verbessertes Verfahren zur Herstellung von 2,3,4,5-Tetrachlorbenzotrifluorid aus 4-Chlorbenzotrifluorid.

2,3,4,5-Tetrachlorbenzotrifluorid ist ein Zwischenprodukt für die Herstellung hochwirksamer antibakterieller Chinoloncarbonsäurederivate (siehe z.B. US-A-5 599 980).

Über eine kinetische Untersuchung der Chlorierung von 4-Chlorbenzotrifluorid mit Chlor in Gegenwart von Eisen wird in Zh. Org. Khimii 20, 2187-2191 (1984) berichtet. Demnach wird als Tetrachlorbenzotrifluorid praktisch nur das 2,3,4,5-Isomer gebildet. Eine präparative Herstellung von 2,3,4,5-Tetrachlorbenzotrifluorid wird hier nicht beschrieben. Im experimentellen Teil dieser Literaturstelle wird eine Chlorierungsgeschwindigkeit von 0,1 g/mol·h angegeben. Für die Herstellung von Tetrachlorbenzotrifluorid würde das eine Chlorierungszeit von über 2000 Stunden bedeuten, was für ein präparatives Verfahren viel zu lange ist.

Ein Herstellungsverfahren für 2,3,4,5-Tetrachlorbenzotrifluorid, bei dem man 4-Chlorbenzotrifluorid mit Chlor in Gegenwart von Eisensulfid als Katalysator umsetzt, ist aus US-A 5 599 980 bekannt. Man erhält dabei das gewünschte Produkt in einer Ausbeute von nur 56,8 % der Theorie, was sehr unbefriedigend ist.

Ein Nachteil aller bisher bekannten Verfahren ist die als Nebenreaktion ablaufende Abspaltung von Fluorwasserstoff. Diese führt zu Korrosionsproblemen und beeinträchtigt die Qualität der gebildeten Chlorwasserstoffsäure. Bei der Herstellung von 3,4-Dichlorbenzotrifluorid, einer Zwischenstufe bei der Herstellung von 2,3,4,5-Tetrachlorbenzotrifluorid aus 4-Chlorbenzotrifluorid, konnte dieses Problem durch Zusatz von Calziumchlorid zum Eisen(III)-chlorid-Katalysator gelöst werden (siehe US-A-4 401 623). Ein derartiges Verfahren ist jedoch nicht auf die Herstellung von 2,3,4,5-Tetrachlorbenzotrifluorid übertragbar, denn die Absorption von Fluorwasserstoff durch Calciumchlorid ist reversibel (siehe a.a.O, Spalte 3, Zeilen 14ff). Für die Herstellung von 2,3,4,5-Tetrachlorbenzotrifluorid werden höhere Temperaturen benötigt als für die Herstellung von 3,4-Dichlorbenzotrifluorid, was die Desorption von Fluorwasserstoff begünstigt. Bei der für die Herstellung von 2,3,4,5-Tetrachlorbenzotrifluorid benötigte Mindesttemperatur von z.B. 80°C ist gemäß der US-A-4 401 623 die Konosionsrate bereits um den Faktor 5 höher als bei der dort sonst angewendeten Temperatur von 60°C (siehe insbesondere Spalte 4, Tabelle II).

Es besteht also noch ein Bedürfnis nach einem Verfahren zur Herstellung von 2,3,4,5-Tetrachlorbenzotrifluorid, bei dem kürzere Reaktionszeiten, bessere Ausbeuten und erniedrigte Gehalte von Fluorwasserstoff im Reaktionsgemisch und im Abgas und damit eine Verbesserung der Korrosionsrate möglich sind.

Es wurde nun ein Verfahren zur Herstellung von 2,3,4,5-Tetrachlorbenzotrifluorid durch Chlorierung von 4-Chlorbenzotrifluorid mit elementarem Chlor gefunden, das dadurch gekennzeichnet ist, dass man die Chlorierung in Gegenwart von 0,1 bis 2 Gew.-% Eisen(III)-Chlorid und 0,05 bis 0,75 Gew.-% Aluminium(III)-Chlorid, jeweils bezogen auf 4-Chlorbenzotrifluorid, durchführt, wobei das Gewichtsverhältnis von Eisen(III)-Chlorid zu Aluminium(III)-Chlorid bei 10:1 bis 1:1 liegt.

Das erfindungsgemäße Verfahren kann man z.B. bei Temperaruten im Bereich 80 bis 150°C durchführen. Vorzugsweise arbeitet man bei 100 bis 130°C.

Der Druck ist beim erfindungsgemäßen Verfahren nicht kritisch. Es kann bei Unterdruck, Überdruck oder Normaldruck gearbeitet werden. Vorzugsweise arbeitet man bei Normaldruck oder bei Drucken bis 1,5 bar.

Man kann das erfindungsgemäße Verfahren mit oder ohne Zusätzen von Lösungsmitteln durchführen. Als Lösungsmittel kommen beispielsweise halogenierte Kohlenwasserstoffe infrage. Vorzugsweise arbeitet man ohne Lösungsmittelzusätze, also in Substanz.

Die für das erfindungsgemäße Verfahren benötigten Reagenzien und Hilfsmittel können in den handelsüblichen Qualitäten eingesetzt werden. Der Wassergehalt des 4-Chlorbenzotrifluorids beträgt vorzugsweise unter 100 ppm, der Wassergehalt des Chlors vorzugsweise unter 50 ppm. Eisen(III)-Chlorid und Aluminium(III)-Chlorid können mit den niedrigen Wassergehalten eingesetzt werden, mit denen sie im Handel erhältlich sind.

Vorzugsweise setzt man, bezogen auf 4-Chlorbenzotrifluorid, 0,25 bis 1 Gew.-% Eisen(III)-Chlorid und 0,1 bis 0,3 Gew.-% Aluminium(III)-Chlorid ein, wobei das Gewichtsverhältnis von Eisen(III)-Chlorid zu Aluminium(III)-Chlorid 5 bis 1,5:1 beträgt.

Man kann das erfindungsgemäße Verfahren in verschiedener Weise durchführen, z.B. diskontinuierlich, kontinuierlich oder teilkontinuierlich in Schüben. Als Beispiel kann man bei diskontinuierlicher Arbeitsweise z.B. 4-Chlorbenzotrifluorid vorlegen, Eisen(III)-Chlorid und Aluminium(III)-Chlorid hinzufügen, das Gemisch unter Rühren auf Reaktionstemperatur erhitzen und mit Chlor solange begasen, bis der gewünschte Umsatz erreicht ist. Man kann die Begasung mit Chlor beispielsweise dann beenden, wenn der Gehalt des Reaktionsgemisches an 2,3,4,5-Tetrachlorbenzotrifluorid 70 bis 80 Gew.-% beträgt. Aus dem Reaktionsgemisch kann man dann das gewünschte Produkt beispielsweise durch Destillation isolieren. Chlorbenzotrifluoride mit niedrigerem Chlorierungsgrad fallen dabei als Vorlauf an und können in die Chlorierung zurückgeführt werden.

Das erfindungsgemäße Verfahren gestattet die Herstellung von 2,3,4,5-Tetrachlorbenzotrifluorid in kürzeren Reaktionszeiten und mit besseren Ausbeuten. Dies ist überraschend, denn häufig sind Eisen(III)-Chlorid und Aluminium(III)-Chlorid gleichwertige Lewis-Säure-Katalysatoren. Hier wirken sie jedoch überraschend in synergistischer Weise zusammen.

Weiterhin ist überraschend, dass der erfindungsgemäße Einsatz von Aluminium(III)-Chlorid zu stark verminderten Gehalten von Fluorwasserstoff im Reaktionsgemisch und im Abgas führt. Aus US-A-5 599 980 (siehe Spalte 7, Zeile 64 bis Spalte 8, Zeile 9) ist bekannt, dass man aus Tetrafluorbenzotrifluorid schon bei 40°C mit Aluminium(III)-Chlorid Tetrafluorbenzotrichlorid herstellen kann. Aufgrund diesen Standes der Technik war also in Gegenwart von Aluminium(III)-chlorid mit erhöhten Fluorwasserstoffgehalten im Reaktionsgemisch und im Abgas und dadurch verursachten größeren Korrosionsproblemen zu rechnen. Überraschenderweise treten beim erfindungsgemäßen Verfahren nur geringe Fluorwasserstoffgehalte im Reaktionsgemisch und im Abgas auf.

Schließlich kann man beim erfindungsgemäßen Verfahren aus dem Abgas eine Salzsäure gewinnen, die allgemein verwendbar ist, während man bei erhöhten Fluorwasserstoffgehalten aus dem Abgas nur eine fluorwasserstoffhaltige Salzsäure gewinnen kann, die wegen ihres hohen Korrosionspotentials nur für spezielle Zwecke verwendbar ist.

### Beispiele

### Beispiel 1

100 Gew.-Teile 4-Chlorbenzotrifluorid wurden in einem Rührgefäß vorgelegt. Dazu wurden 0,5 Gew.-Teile Eisen(III)-Chlorid und 0,167 Gew.-Teile Aluminium(III)-Chlorid gegeben. Unter Rühren erhitzte man auf 110°C und leitete unter Rühren 166 Gew.-Teile Chlor im Verlaufe von 117 Stunden ein. Nach 3-stündigem Ausblasen mit Stickstoff erhielt man 157 Gew.-Teile eines Reaktionsgemisches folgender gaschromatografisch ermittelter Zusammensetzung: 0,8 Gew.% Dichlorbenzotrifluoride, 13,5 Gew.-% Trichlorbenzotrifluoride, 74,1 Gew.-% 2,3,4,5-Tetrachlorbenzotrifluorid und 8,0 Gew.-% Pentachlorbenzotrifluorid. Das entspricht eine Ausbeute an 2,3,4,5-Tetrachlorbenzotrifluorid von 74,0 % der Theorie. Das Abgas wurde auf den Gehalt an Fluorwasserstoff analysiert. Er betrug zwischen der 80. Stunde und dem Ende der Chlorierung weniger als 5 ppm. Die Dichlor- und Trichlorbenzotrifluoride, die mit einer Ausbeute von 1,1 bzw. 15,3 % der Theorie anfielen, sind nach destillativer Abtrennung rückführbar.

### Beispiel 2 (zum Vergleich Arbeitsweise nur mit Eisen(III)-Chlorid als Katalysator)

100 Gew.-Teile 4-Chlorbenzotrifluorid wurden in einem Rührgefäß vorgelegt. Dazu wurden 0,5 Gew.-Teile Eisen(III)-Chlorid gegeben. Unter Rühren erhitzte man auf 110°C und leitete 166 Gew.-Teile Chlor so ein, dass eine Produktzusammensetzung anfiel, die derjenigen des Beispiels 1 möglichst ähnlich war, was 126 Stunden benötigte. Nach 3-stündigem Ausblasen mit Stickstoff erhielt man 150 Gew.-Teile eines Reaktionsgemisches folgender gaschromatografisch ermittelter Zusammensetzung: 1,2 Gew.-% Dichlorbenzotrifluoride, 13,8 Gew.-% Trichlorbenzotrifluoride, 73,4 Gew.-% 2,3,4,5-Tetrachlorbenzotrifluorid und 7,1 Gew.-% Pentachlorbenzotrifluorid. Das entspricht einer Ausbeute von 70,1 % der Theorie. Das Abgas wurde auf den Gehalt an Fluorwasserstoff analysiert. Er betrug zwischen der 80. Stunde und dem Ende der Chlorierung zwischen 40 und 50 ppm.

## Patentansprüche

1. Verfahren zur Herstellung von 2,3,4,5-Tetrachlorbenzotrifluorid durch Chlorierung von 4-Chlorbenzotrifluorid mit elementarem Chlor, **dadurch gekennzeichnet, dass** man die Chlorierung in Gegenwart von 0,1 bis 2 Gew.-% Eisen(III)-Chlorid und 0,05 bis 0,75 Gew.-% Aluminium(III)-Chlorid jeweils bezogen auf 4-Chlorbenzotrifluorid, durchführt, wobei das Gewichtsverhältnis von Eisen(III)-Chlorid zu Aluminium(III)-Chlorid bei 10:1 bis 1:1 liegt.

2. Verfahren nach Anspruch 1, **dadurch gekennzeichnet, dass** man es bei Temperaturen im Bereich 80 bis 150°C durchführt.

3. Verfahren nach Ansprüchen 1 und 2, **dadurch gekennzeichnet, dass** man es ohne Lösungsmittelzusätze durchführt.

4. Verfahren nach Ansprüchen 1 bis 3, **dadurch gekennzeichnet, dass** der Wassergehalt des eingesetzten 4-Chlorbenzotrifluorids unter 100 ppm und der Wassergehalt des eingesetzten Chlors unter 50 ppm beträgt.

5. Verfahren nach Ansprüchen 1 bis 4, **dadurch gekennzeichnet, dass** man, bezogen auf 4-Chlorbenzotrifluorid, 0,25 bis 1 Gew.-% Eisen(III)-Chlorid und 0,1 bis 0,3 Gew.-% Aluminium(III)-Chlorid einsetzt, wobei das Gewichtsverhältnis Eisen(III)-Chlorid zu Aluminium(III)-Chlorid 5 bis 1,5:1 beträgt.

6. Verfahren nach Ansprüchen 1 bis 5, **dadurch gekennzeichnet, dass** man es diskontinuierlich, kontinuierlich oder teilkontinuierlich in Schüben durchführt.

## Claims

1. Method for producing 2,3,4,5-tetrachlorobenzotrifluoride by chlorinating 4-chlorobenzotrifluoride with elemental chlorine, **characterized in that** the chlorination is carried out in the presence of from 0.1 to 2% by weight of iron(III) chloride and 0.05 to 0.75% by weight of aluminum(III) chloride, in each case based on 4-chlorobenzotrifluoride, where the weight ratio of iron(III) chloride to aluminum(III) chloride is 10:1 to 1:1.

2. Method according to Claim 1, **characterized in that** it is carried out at temperatures in the range 80 to 150°C.

3. Method according to Claims 1 and 2, **characterized in that** it is carried out without solvent additions.

4. Method according to Claims 1 to 3, **characterized in that** the water content of the 4-chlorobenzotrifluoride used is less than 100 ppm and the water content of the chlorine used is less than 50 ppm.

5. Method according to Claims 1 to 4, **characterized in that**, based on 4-chlorobenzotrifluoride, 0.25 to 1% by weight of iron(III) chloride and 0.1 to 0.3% by weight of aluminum(III) chloride are used, where the weight ratio of iron(III) chloride to aluminum(III) chloride is 5 to 1.5:1.

6. Method according to Claims 1 to 5, **characterized in that** it is carried out discontinuously, continuously or semicontinuously in pulses.

## Revendications

1. Procédé pour la préparation du 2,3,4,5-tétrachlorobenzotrifluorure par chloruration du 4-chlorobenzotrifluorure à l'aide du chlore élémentaire, **caractérisé en ce que** l'on procède à la chloruration en présence de 0,1 à 2 % en poids de chlorure de fer (III) et 0,05 à 0,75 % en poids de chlorure d'aluminium (III), dans les deux cas par rapport au 4-chlorobenzotrifluorure, avec des proportions relatives en poids de 10:1 à 1:1 entre le chlorure de fer (III) et le chlorure d'aluminium (III).

2. Procédé selon la revendication 1, **caractérisé en ce que** l'on opère à des températures dans l'intervalle de 80 à 150°C.

3. Procédé selon la revendication 1 et 2, **caractérisé en ce que** l'on opère sans adjonction de solvant.

4. Procédé selon les revendications 1 à 3, **caractérisé en ce que** la teneur en humidité du 4-chlorobenzotrifluorure mis en oeuvre est inférieure à 100 ppm et la teneur en humidité du chlore mis en oeuvre est inférieure à 50 ppm.

5. Procédé selon les revendications 1 à 4, **caractérisé en ce que** l'on utilise de 0,25 à 1 % en poids de chlorure de fer (III) et 0,1 à 0,3 % en poids de chlorure d'aluminium (III), dans les deux cas par rapport au 4-chlorobenzotrifluorure, avec des proportions relatives en poids de 5 à 1,5:1 entre le chlorure de fer (III) et le chlorure d'aluminium (III).

6. Procédé selon les revendications 1 à 5, **caractérisé en ce que** l'on opère en discontinu, en continu ou en partiellement continu, par fournées.
